(19) Europäisches Patentamt
European Patent Office
Office européen des brevets

(11) Veröffentlichungsnummer: **0 002 526**
**B1**

(12) # EUROPÄISCHE PATENTSCHRIFT

(45) Veröffentlichungstag der Patentschrift: **02.09.81**

(51) Int. Cl.³: **C 07 C 127/00**

(21) Anmeldenummer: **78101678.7**

(22) Anmeldetag: **14.12.78**

(54) **Verfahren zur gleichzeitigen Herstellung von 1,3-disubstituierten Harnstoffen und 1,2-Diolen.**

(30) Priorität: **17.12.77 DE 2756409**

(43) Veröffentlichungstag der Anmeldung: **27.06.79 Patentblatt 79/13**

(45) Bekanntmachung des Hinweises auf die Patenterteilung: **02.09.81 Patentblatt 81/35**

(84) Benannte Vertragsstaaten: **BE CH DE FR GB**

(56) Entgegenhaltungen:
**DE-A-848 039**
**DE-A-2 354 952**
**FR-A-1 096 204**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Dessauer Weg 6, D-6800 Mannheim (DE)**
Erfinder: **Fischer, Karl, Dr., Luginsland 22, D-6520 Worms (DE)**
Erfinder: **Woerz, Otto, Dr., Bruesseler Ring 51, D-6700 Ludwigshafen (DE)**

**EP 0 002 526 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur gleichzeitigen Herstellung von 1,3-disubstituierten Harnstoffen und 1,2-Diolen

Die Erfindung betrifft ein Verfahren zur gleichzeitigen Herstellung von 1,3-disubstituierten Harnstoffen und 1,2-Diolen durch Umsetzung von cyclischen Carbonaten mit Aminen in bestimmtem Mengenverhältnis bei höherer Temperatur.

Glykole wie Äthylenglykol können beispielsweise durch alkalische Verseifung von Dichlorverbindungen oder Chlorhydrinen, z. B. Äthylenchlorhydrin oder 1,2-Dichloräthan, hergestellt werden (Ullmanns Encyclopädie der technischen Chemie, 4. Auflage, Band 8, Seite 201). Die stark korrodierenden Eigenschaften dieser Reaktionsgemische standen jedoch ihrer Handhabung in großtechnischen Anlagen entgegen. In neuerer Zeit gewann daher auch die säurekatalysierte Hydrolyse von Äthylenoxid größere wirtschaftliche Bedeutung. Ein Nachteil sind hier die konkurrierende Bildung von Polyglykolen sowie der stark exotherme Charakter der Ringöffnung.

Als ein industriell wichtiges Verfahren zur Herstellung von Harnstoffen ist vor allem die Umsetzung von Isocyanaten, Carbaminsäurechloriden oder Phosgen mit Aminen zu nennen (Ullmann, 3. Auflage, Band 8, Seite 390). Ihr Nachteil besteht in der Handhabung sehr toxischer und umweltbelastender Stoffe wie Phosgen und Salzsäure. Ferner ist ihre Wärmetönung nur schwer technisch zu beherrschen, und die entstehenden Harnstoffe sind mit salzartigen Verbindungen verunreinigt. Die Umamidierung von Harnstoff benötigt sehr hohe Überschüsse an Aminen, um zu befriedigenden Ausbeuten zu gelangen; Wegen dem Vorliegen eines Gleichgewichts sind die Endprodukte stets mit wechselnden Mengen halbseitig substituierte Harnstoffe verunreinigt. Nachteilig ist auch, daß gasförmiges Ammoniak frei wird und entweder verbrannt oder durch Neutralisation mit wäßriger Salzsäure gebunden werden muß.

Alle genannten Verfahren sind daher für einen großtechnischen Einsatz vom Standpunkt der Wirtschaftlichkeit und Umweltfreundlichkeit nicht befriedigend.

Die französische Patentschrift 1 096 204 beschreibt, daß bei der Umsetzung von Alkylencarbonaten mit Aminen nicht 2 Bruchstücke — substituierter Harnstoff und Glykol — entstehen, sondern daß sich der Ring unter Bildung nur eines substituierten Carbamates, z. B. des $\beta$-Hydroxyäthylcarbamates, öffnet. Weiterhin ist diese Carbamatbildung unabhängig von der eingesetzten Aminmenge; selbst mit überschüssigem Amin, wie es in der genannten Patentschrift empfohlen wird, erhält man keine Harnstoffe. Nach der gleichen Arbeitsweise wird ferner die Einhaltung niedriger Temperaturen (0 bis 50° C) gefordert, um zu befriedigenden Ausbeuten zu gelangen. Bei Verwendung von Wasser muß bei 0 bis 10° C gearbeitet werden, da sonst das Amin eine verseifende Wirkung, z. B. wie Alkali, ausübt und zur Hydrolyse in Glykol und Amin, jedoch zu keinem Harnstoff führt. Beispiel 4 zeigt deutlich an der Umsetzung des Glykolcarbonats mit Propylamin bei 40° C die Urethanbildung (96% Ausbeute).

Die Empfindlichkeit der gleichen Carbamate beschreibt auch eine Arbeit in Bull. Soc. chim. France (Band 1956, Seiten 831 bis 836), die feststellt, daß $\beta$-Hydroxyäthylcarbamate sich nicht einmal unter reduziertem Druck destillieren lassen. Als Zersetzungsprodukt werden aus 2 Molekülen $\beta$-Hydroxyäthylcarbamat, Glykol, Kohlendioxid, Äthylenoxid und 1 Molekül Harnstoff gewonnen.

Im Falle aromatischer Amine führte die Umsetzung mit Äthylencarbonat je nach Temperaturbereich unter Decarboxylierung zu Hydroxyäthylaminen (150 bis 190° C) oder zu 3-Aryloxazolidon-(2)-en (180 bis 200° C), wie jüngere Untersuchungen von E. Gulbins und K. Hamann zeigen (Chem. Ber., Band 99, Seiten 55 bis 61 (1966)).

Weiterhin zeigt DE-A-848 039 die Umsetzung von neutralen Kohlensäureestern von hydroxylhaltigen Verbindungen mit primären aliphatischen Aminen bei 20—70° C, wobei symmetrischer Dialkylharnstoff in fast quantitativer Ausbeute entsteht.

DE-A-2 354 952 zeigt in Beispiel 3 die Umsetzung von Äthylencarbonat mit 3-Dimethylaminopropylamin während 17 Stunden bei 140° C, wobei man in 90%iger Ausbeute N,N'-Bis(3-dimethylaminopropyl)-harnstoff erhält.

Es wurde nun gefunden, daß man gleichzeitig 1,3-disubstituierte Harnstoffe der Formel

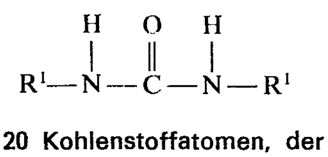

(I)

worin $R^1$ einen Alkylrest mit 1 bis 20 Kohlenstoffatomen, der durch Phenoxygruppen und/oder Alkoxygruppen mit 1 bis 13 Kohlenstoffatomen substituiert sein kann, einen Monocycloalkylrest mit 3 bis 8 Kohlenstoffatomen, einen Bicycloalkylrest mit 6 bis 12 Kohlenstoffatomen oder einen Aralkylrest mit 7 bis 20 Kohlenstoffatomen bedeutet, und 1,2-Diole der Formel

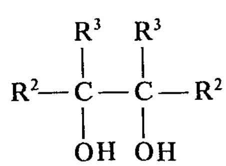

(II)

worin die einzelnen Reste $R^2$ und $R^3$ gleich oder verschieden sind und jeweils ein Wasserstoffatom oder einen aliphatischen Rest bezeichnen, das Restpaar $R^3$ auch zusammen mit den beiden benachbarten Kohlenstoffatomen für Glieder eines Ringes stehen können, vorteilhaft erhält, wenn man cyclische, aliphatische Carbonate der Formel

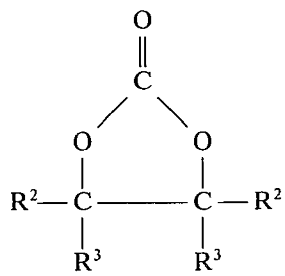

(III)

worin $R^2$ und $R^3$ die vorgenannte Bedeutung besitzen, mit Aminen der Formel

$$R^1—NH_2 \qquad (IV)$$

worin $R^1$ die vorgenannte Bedeutung besitzt, in einem Verhältnis von 2 bis 20 Mol Ausgangsstoff IV je Mol Ausgangsstoff III bei einer Temperatur oberhalb 100° C umsetzt.

Die Umsetzung läßt sich für den Fall der Verwendung von Äthylencarbonat und n-Propylamin durch die folgende Formelgleichung wiedergeben:

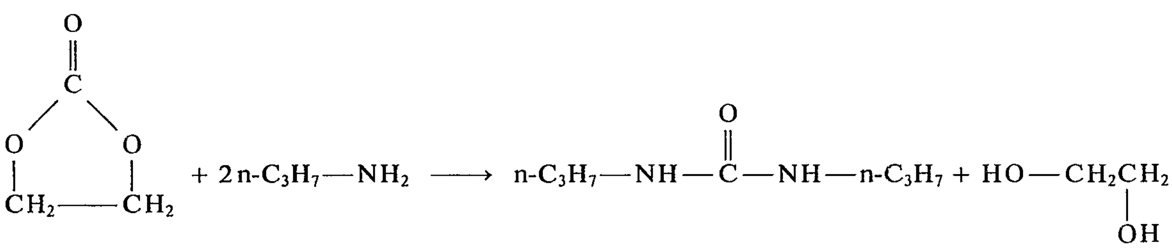

Im Vergleich zu den bekannten Verfahren liefert das Verfahren nach der Erfindung auf einfacherem und wirtschaftlicherem Wege gleichzeitig 1,3-disubstituierte Harnstoffe und 1,2-Diole in guter Ausbeute und Reinheit und somit insgesamt in besserer Raum-Zeit-Ausbeute. Die Reaktionszeit ist im allgemeinen kurz, die Aufarbeitung des Reaktionsgemisches, gerade auch im Hinblick auf den Umweltschutz einfacher und betriebssicherer. Alle diese vorteilhaften Ergebnisse sind im Hinblick auf den Stand der Technik überraschend. Es mußte insbesondere erwartet werden, daß die erfindungsgemäßen Temperaturen und Aminmengen eine Bildung von Harnstoffen nicht erlauben bzw. zumindest heterogene Gemische von Zersetzungs- und Kondensationsprodukten sowie Spaltprodukten des cyclischen Carbonats liefern. Im Gegensatz zu den bekannten Verfahren werden Harnstoff und Glykol überraschend gleichzeitig in hoher Reinheit und in praktisch quatitativer Ausbeute erhalten.

Bevorzugte Ausgangsstoffe III und IV und dementsprechend bevorzugte Endstoffe I und II sind solche, in deren Formeln $R^1$ einen Alkylrest mit 1 bis 20, insbesondere 1 bis 8 Kohlenstoffatomen, einen Monocycloalkylrest mit 3 bis 8 Kohlenstoffatomen, einen Bicycloalkylrest mit 6 bis 12 Kohlenstoffatomen, einen Aralkylrest mit 7 bis 20, insbesondere 7 bis 12 Kohlenstoffatomen, einen durch mehrere Phenoxygruppen oder insbesondere durch eine Phenoxygruppe und/oder durch mehrere Alkoxygruppen oder insbesondere durch eine Alkoxygruppe mit 1 bis 13 Kohlenstoffatomen substituierten Alkylrest mit 1 bis 20, bevorzugt 2 bis 20, insbesondere 2 bis 8 Kohlenstoffatomen, bedeutet, die einzelnen Reste $R^2$ und $R^3$ gleich oder verschieden sein können und jeweils ein Wasserstoffatom oder einen Alkylrest mit 1 bis 5 Kohlenstoffatomen bezeichnen, das Restpaar $R^3$ auch zusammen mit den beiden benachbarten Kohlenstoffatomen für Glieder eines cycloaliphatischen Ringes, insbesondere eines Cycloalkylringes mit 5 bis 8 Kohlenstoffatomen stehen können.

Bevorzugt kommen als Ausgangsstoffe III in Betracht: Äthylencarbonat, Propylencarbonat, 1,2-Butylencarbonat, 2,3-Butylencarbonat, 1,2-Pentylencarbonat, 2,3-Pentylencarbonat, 3-Methyl-1,2-butylencarbonat, 2,2-Dimethyläthylencarbonat, 2-Methyl-2,3-butylencarbonat, 1,2-Hexylencarbonat, 2,3-Hexylencarbonat, 3,4-Hexylencarbonat, Cyclopentylencarbonat, Cyclohexylencarbonat, Cycloheptylencarbonat, Cyclooctylencarbonat, 3-Äthyl-3,4-butylencarbonat, 2-Methyl-1,2-pentylencarbonat.

Als Ausgangsstoffe IV kommen bevorzugt in Betracht: Methylamin, Äthylamin, n-Propylamin, Isopropylamin, Cyclopropylamin, n-Butylamin, Isobutylamin, sek.-Butylamin, tert.-Butylamin, Cyclobuamin, 2-Methylbutylamin-2, n-Hexylamin, Hexylamin-2, Hexylamin-3, 3-Methylpentylamin-2, 4-Methyltylamin, n-Pentylamin, Pentylamin-2, Pentylamin-3, 3-Methylbutylamin-2, Neopentylamin, Cyclopentyl-

pentylamin-2,2,2-Dimethylbutylamin-1, 2-Methylpentylamin-2, Cyclohexylamin, n-Heptylamin, Heptylamin-2, Heptylamin-3, Heptylamin-4, 3-Methylhexylamin-2, 4-Methylhexylamin-2, 5-Methylhexylamin-2, 2,2-Dimethylpentylamin, 3,3-Dimethylpentylamin-2, 2-Methylheptylamin-2, Cycloheptylamin, n-Octylamin, Octylamin-2, Octylamin-3, Octylamin-4, 3-Methyloctylamin-2, 4-Methyloctylamin-2, 5-Methyloctylamin-2, 2,2-Dimethylhexylamin-1, Cyclooctylamin, 3-Methylheptylamin-2, 4-Methylheptylamin-2, 5-Methylheptylamin-2, n-Nonylamin, n-Decylamin, n-Undecylamin, n-Dodecylamin, n-Tridecylamin, n-Tetradecylamin, n-Pentadecylamin, n-Hexadecylamin, n-Heptadecylamin, n-Octadecylamin, n-Nonadecylamin, Eicosylamin, Methoxy-(1)-äthylamin-2, Methoxy-(1)-propylamin-2, Methoxy-(1)-propylamin-3, Methoxy-(1)-butylamin-2, Methoxy-(1)-butylamin-3, Methoxy-(1)-butylamin-4, Methoxy-(1)-pentylamin-5, Methoxy-(1)-hexylamin-6, Methoxy-(1)-heptylamin-7, Methoxy-(1)-octylamin-8, Methoxy-(1)-nonylamin-9, Methoxy-(1)-decylamin-10, Äthoxy-(1)-äthylamin-2, Äthoxy-(1)-propylamin-3, Äthoxy-(1)-butylamin-4, Äthoxy-(1)-pentylamin-5, Äthoxy-(1)-hexylamin-6, Äthoxy-(1)-heptylamin-7, Äthoxy-(1)-octylamin-8, Äthoxy-(1)-nonylamin-9, Äthoxy-(1)-decylamin-10, n-Propoxy-(1)-äthylamin-2, n-Butoxy-(1)-äthylamin-2, n-Pentoxy-(1)-äthylamin-2, n-Hexoxy-(1)-äthylamin-2, n-Heptoxy-(1)-äthylamin-2, n-Octoxy-(1)-äthylamin-2, n-Nonoxy-(1)-äthylamin-2, n-Decyloxy-(1)-äthylamin-2, n-Undecycloxy-(1)-äthylamin-2, Dodecycloxy-(1)-äthylamin-2, Tridecycloxy-(1)-äthylamin-2, Benzylamin, $\alpha$-Methylbenzylamin, 2-Phenyläthylamin, Phenyl-(3)-propylamin-1, Phenyl-(3)-propylamin-2, Phenyl-(3)-propylamin-3, Phenyl-(4)-butylamin-2, Isoproxy-(1)-äthylamin-2, Isopropoxy-(1)-propylamin-2, Isopropoxy-(1)-propylamin-3, Isopropoxy-(1)-butylamin-3, Phenoxy-(1)-äthylamin-2, Phenoxy-(1)-propylamin-2, Phenoxy-(1)-propylamin-3, Phenoxy-(1)-butylamin-4 und Norbornylamin-2.

Der Ausgangsstoff III wird mit dem Ausgangsstoff IV in einem Verhältnis von 2 bis 20, vorzugsweise 4 bis 10 Mol Ausgangsstoff IV je Mol Ausgangsstoff III umgesetzt. Die Umsetzung wird bei einer Temperatur oberhalb 100°C, zweckmäßig von 105 bis 250°C, vorzugsweise von 140 bis 240°C, insbesondere von 170 bis 220°C, drucklos oder unter Druck, kontinuierlich oder diskontinuierlich durchgeführt. Man kann das eingesetzte Amin IV gleichzeitig als Lösungsmittel für das meist kristalline Carbonat III verwenden. Gegebenenfalls kann die Reaktion jedoch auch in Gegenwart eines unter den Reaktionsbedingungen inerten, organischen Lösungsmittels durchgeführt werden. Hierzu eignen sich aliphatische, aromatische oder araliphatische Kohlenwasserstoffe wie Pentan, Hexan, Cyclohexan, Heptan, Pinan, Nonan, o-, m-, p-Cymol, Benzinfraktionen innerhalb eines Siedepunktes von 70 bis 190°C, Methylcyclohexan, Dekalin, Petroläther, Ligroin, 2,2,4-Trimethylpentan, 2,2,3-Trimethylpentan, 2,3,3-Trimethylpentan, Octan, Benzol, Toluol, Xylol, Naphthalin, Tetralin; Äther, z. B. Diäthyläther, Äthylpropyläther, Methyl-tert.-butyläther, n-Butyläther, Di-n-butyläther, Diisobutyläther, Diisoamyläther, Diisopropyläther, Anisol, Phenetol, Cyclohexylmethyläther, Äthylenglykoldimethyläther, Tetrahydrofuran, Dioxan, Thioanisol; Alkohole, z. B. Amylalkohol, n-Butanol, sek.-Butanol, Isobutanol, tert.-Butylalkohol, Isopropanol, n-Propanol, Äthanol, Methanol, Glykol, Cyclohexanol, Heptanol, Octanol; und entsprechende Gemische. Das Lösungsmittel wird zweckmäßig in einer Menge von 50 bis 10 000, vorzugsweise von 200 bis 1000 Gewichtsprozent Lösungsmittel, bezogen auf Ausgangsstoff III, verwendet.

Die Reaktion wird wie folgt durchgeführt: Ein Gemisch von Ausgangsstoff III und IV, gegebenenfalls zusammen mit Lösungsmittel, wird während 0,3 bis 6 Stunden bei der Reaktionstemperatur und im erfindungsgemäßen Molverhältnis umgesetzt. Die Reihenfolge der Zugabe ist beliebig. In einer bevorzugten Ausführungsform des erfindungsgemäßen Verfahrens läßt man bei Raumtemperatur ein Carbonat III zu einem Amin IV zulaufen und setzt dann bei der Reaktionstemperatur um. Wegen des hohen Dampfdruckes niedrigsiedender Amine vermischt man die Komponenten zweckmäßig bei Raumtemperatur und erhöht erst nach Verschließen des Reaktionsgefäßes auf die Umsetzungstemperatur. Hierzu ist bei Aminen niederen Molekulargewichtes die Verwendung eines Druckbehälters erforderlich. Bei höhersiedenden Aminen läßt sich dagegen die Umsetzung drucklos im offenen System oder unter Rückfluß durchführen. In diesem Falle kann das entstehende 1,2-Diol II auch kontinuierlich aus dem Reaktionsgemisch abdestilliert werden.

Bei Verwendung geeigneter Druckdosierungsvorrichtungen lassen sich die Ausgangsstoffe III und IV auch von vornherein gleich bei der Reaktionstemperatur zugeben und umsetzen. Nach der Reaktion wird der Endstoff I und II aus dem Gemisch in üblicher Weise, z. B. durch Filtration und fraktionierte Destillation, isoliert. Zur Aufarbeitung saugt man zweckmäßig die meist in kristalliner Form anfallenden Harnstoffe I ab und unterwirft das Filtrat einer fraktionierten Destillation. Als niedrig siedende Komponente fallen hierbei, je nach Molekulargewicht, gewöhnlich zunächst die im Überschuß eingesetzten Ausgangsamine IV oder das gegebenenfalls verwendete Lösungsmittel an; bei höherer Temperatur gehen dann die Endstoffe II über, wobei meist eine geringe Menge spurenweise gelösten reinen Harnstoffes I zurückbleibt.

Bei ölig anfallenden Harnstoffen I unterwirft man zweckmäßig gleich das gesamte Reaktionsgemisch einer fraktionierten Destillation, wobei zunächst das überschüssige Ausgangsamin IV und dann als höhersiedende Kompoenten das Diol übergeht; hierbei verbleibt als Rückstand der reine Harnstoff I.

Man kann jedoch auch im Falle wasserlöslicher Amine IV das anfallende Reaktionsgemisch mit Wasser versetzen, den unlöslichen Harnstoff I absaugen und aus dem Filtrat durch fraktionierte Destillation die reinen Ausgangsamine IV und Diole II zurückgewinnen.

Die nach dem erfindungsgemäßen Verfahren herstellbaren Harnstoffe I sind wertvolle Ausgangsmaterialien zur Synthese von Farbstoffen und Pflanzenschutzmitteln. So erhält man aus Harnstoffen bei der Einwirkung von Oleum nach dem Verfahren der englischen Patentschrift 1 185 439 oder mit Schwefeltrioxid und dann mit Schwefelsäure nach der deutschen Offenlegungsschrift 2 424 371 Amidosulfonsäuren.

Die nach diesem Verfahren herstellbaren Verbindungen sind Süßstoffe, insbesondere die Cyclohexylamidosulfonsäure bzw. deren Calcium-, Natrium- und Kaliumsalze, und wertvolle Ausgangsstoffe für die Herstellung von Süßstoffen, Farbstoffen und Schädlingsbekämpfungsmitteln. Beispielsweise können durch Chlorierung, z. B. mit Thionylchlorid, die entsprechenden Sulfonsäurechloride, z. B. Isopropylaminosulfonylchlorid, hergestellt werden: aus ihnen kann man durch Umsetzung mit Anthtanilsäure oder ihren Salzen o-Sulfamidobenzoesäuren herstellen. Durch Cyclisierung dieser Stoffe gelangt man zu den 2,1,3-Benzothiadiazin-4-on-2,2-dioxiden, deren Verwendung für Pflanzenschutzmittel und Pharmazeutika in der deutschen Offenlegungsschrift 2 105 687 beschrieben ist. Bezüglich der Verwendung wird auf die vorgenannte Veröffentlichung und auf DAS 1 120 456, DP 1 242 627 und DOS 1 542 836 verwiesen. Die Umsetzung der Amidosulfonsäuren mit halogenierenden Agentien nach dem Verfahren der US-Patentschrift 3 992 444 liefern Alkylamidosulfonylhalogenide, deren Verwendung zur Synthese von Farbstoffe und Pflanzenschutzmitteln in der gleichen Patentschrift beschrieben wird; weitere Anwendungsmöglichkeiten als Vorprodukte für Herbizide sind in den deutschen Offenlegungsschriften 2 201 432 und 2 310 757 beschrieben.

Die nach dem erfindungsgemäßen Verfahren weiterhin anfallenden 1,2-Diole II sind wertvolle Lösungsmittel und Zwischenprodukte für die Herstellung von Farbstoffen und Pflanzenschutzmitteln. Beispielsweise wird Äthylenglykol in großem Maßstab als Frostschutzmittel für Verbrennungsmotoren eingesetzt. Weiterhin sind sie Ausgangsstoffe für die Herstellung von Polyesterharzen, Weichmachern, Alkydharzen, Schmierölen, Hydraulikflüssigkeiten, Polyurethanen und Waschrohstoffen. Bezüglich der Verwendung wird auf Ullmanns Encyclopädie der technischen Chemie, Ergänzungsband, Seite 101 bis 103, verwiesen.

Die in den folgenden Beispielen aufgeführten Teile bedeuten Gewichtsteile.

## Beispiel 1

64,9 Teile Äthylencarbonat werden unter Rühren bei 25 bis 30°C in 325 Teile Isopropylamin (Molverhältnis Ausgangsstoff IV zu Ausgangsstoff III: 7,5 : 1) in einem Autoklaven eingeführt. Anschließend wird das Gemisch 3 Stunden bei 180°C gerührt. Nach dem Abkühlen und Absaugen werden 94 Teile N,N'-Diisopropylharnstoff mit Fp 189 bis 190°C erhalten. Aus dem Filtrat wird das überschüssige Isopropylamin abdestilliert und dann der Rückstand im Vakuum destilliert. Dabei werden 43,5 Teile Glykol (=95% der Theorie ) vom Sdp 46—50°C/$_{0,01\ mbar}$ gewonnen. Als Rückstand verbleiben 5 Teile farbloser N,N'-Diisopropylharnstoff mit Fp 188 bis 190°C; Gesamtausbeute 101 Teile (95% der Theorie).

## Beispiel 2

21 Teile Äthylencarbonat werden unter Rühren in 120 Teilen Isopropylamin (Molverhältnis Ausgangsstoff IV zu Ausgangsstoff III: 8,5 : 1) bei Raumtemperatur gelöst und anschließend 4 Stunden bei 170°C gerührt. Das Reaktionsgemisch wird auf 35°C abgekühlt und dann durch Destillation vom überschüssigen Isopropylamin befreit. Nun wird der Rückstand mit 50 Teilen Wasser versetzt und abgesaugt. Nach dem Trocknen werden 33,9 Teile (98,3% der Theorie) N,N'-Diisopropylharnstoff mit Fp 188 bis 190°C erhalten.

Die wässerige Phase enthält gemäß der gaschromatographischen Untersuchung 14,5 Teile (98% der Theorie) Glykol.

## Beispiel 3

121,2 Teile 2-Phenyläthylamin und 18,5 Teile Äthylencarbonat (Molverhältnis Ausgangsstoff IV zu Ausgangsstoff III: 4,8 : 1) werden in ein Bombenrohr eingefüllt und 3 Stunden bei 190°C geschüttelt. Nach dem Abkühlen wird das Reaktionsgemisch abgesaugt, wobei 52,3 Teile N,N'-Di-2-phenyläthylharnstoff mit Fp 134 bis 138°C isoliert werden. Nach Entfernung des Isopropylamins im Vakuum werden durch Destillation des Filtrats 12,4 Teile (94,4% der Theorie) Glykol vom Sdp 46—50°C/$_{0,01\ mbar}$ und als Rückstand nochmals 2 Teile N,N'-Di-2-phenyläthylharnstoff gewonnen; Gesamtausbeute 54,3 Teile (96,2% der Theorie).

Beispiel 4

a) In 128,7 Teile Tridecyloxypropylamin werden unter Rühren bei Raumtemperatur 9,7 Teile Äthylencarbonat (Molverhältnis Ausgangsstoff IV zu Ausgangsstoff III: 4,5 : 1) eingeführt. Nach einer Stunde Erhitzen bei 200° C wird das Gemisch im Vakuum destilliert, wobei 6,52 Teile (95,4% der Theorie) Glykol vom Sdp 46—50° C/$_{0,01\ mbar}$ als Destillat und im Rückstand 59,4 Teile (100% der Theorie) N,N'-Di-tridecyloxypropylharnstoff als farbloses Öl mit $n_D^{25}$ = 1,4672 erhalten werden.

b) Unter gleichen Reaktionsbedingungen, jedoch unter Verwendung von 100 Teilen Cyclohexan als Lösungsmittel, werden 59,4 Teile (100% der Theorie) N,N'-Di-tridecyloxypropylharnstoff in gleicher Reinheit und 6,58 Teile Glykol (96,3% der Theorie) erhalten.

Beispiele 5 bis 14

Analog Beispiel 4a) werden die in der Tabelle aufgeführten Endstoffe erhalten.

| Beispiel | Teile | Ausgangsstoff III | Teile | $R^1NH_2$<br>$R^1$ – | Reaktionszeit (Std.) | Temperatur (°C) | Teile | $R^1NH\overset{O}{\overset{\|}{C}}—NHR^1$<br>$R^1$— | Ausbeute (% der Theorie) | Fp. (°C) | Teile | Diol II | Ausbeute (% der Theorie) |
|---|---|---|---|---|---|---|---|---|---|---|---|---|---|
| 5 | 17,6 | 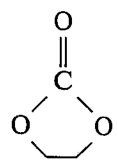 | 43,5 | $CH_3$— | 0,33 | 210 | 17,08 | $CH_3$— | 97 | 100 | 11,8 | $HO-CH_2-CH_2OH$ | 95 |
| 6 | 17,6 | desgl. | 63,1 | $C_2H_5$— | 1 | 190 | 22,3 | $C_2H_5$— | 96 | 108-110 | 11,7 | desgl. | 94 |
| 7 | 17,6 | desgl. | 82,8 | n-$C_3H_7$— | 3 | 180 | 27,86 | n-$C_3H_7$— | 96,6 | 105 | 11,6 | desgl. | 93,4 |
| 8 | 17,6 | desgl. | 87,76 | sek.-$C_4H_9$— | 4 | 170 | 33,1 | sek.-$C_4H_9$— | 96,1 | 135-136 | 11,9 | desgl. | 95,8 |
| 9 | 17,6 | desgl. | 113,3 | $(CH_3)_2-CH-\underset{CH_3}{\underset{\|}{CH}}-$ | 3 | 180 | 38,9 | $(CH_3)_2CH-\underset{CH_3}{\underset{\|}{CH}}-$ | 97,3 | 230-232 | 11,7 | desgl. | 94 |
| 10 | 22,9 | desgl. | 129,3 | $CH_3-O-CH_2-CH_2-$ | 4 | 190 | 42,3 | $CH_3-O-CH_2-CH_2-$ | 92,8 | 59-64 | 15,5 | desgl. | 96 |
| 11 | 18,5 | desgl. | 111 | 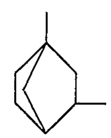 | 4 | 180 | 48,8 | 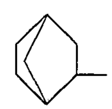 | 94 | 269-276 | 12,4 | desgl. | 96 |
| 12 | 20 | desgl. | 130 | 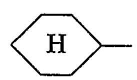 | 3 | 180 | 47,4 | 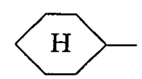 | 93 | 183-184 | 13,3 | desgl. | 94,3 |
| 13 | 13,2 | desgl. | 129,5 | $C_{13}H_{27}$— | 4 | 180 | 63,6 | $C_{13}H_{27}$— | 99,8 | $n_D^{25}=1,4692$ | 9,0 | desgl. | 96,6 |
| 14 | 27,8 | 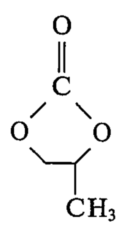 | 120 | i-$C_3H_7$— | 2 | 190 | 35,2 | i-$C_3H_7$— | 90 | 187-190 | 19,3 | $HO-CH_2-\underset{CH_3}{\underset{\|}{CHOH}}$ | 93 |

**Patentanspruch**

Verfahren zur gleichzeitigen Herstellung von 1,3-disubstituierten Harnstoffen der Formel

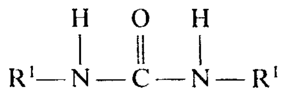

(I)

worin $R^1$ einen Alkylrest mit 1 bis 20 Kohlenstoffatomen, der durch Phenoxygruppen und/oder Alkoxygruppen mit 2 bis 13 Kohlenstoffatomen substituiert sein kann, einen Monocycloalkylrest mit 3 bis 8 Kohlenstoffatomen, einen Bicycloalkylrest mit 6 bis 12 Kohlenstoffatomen oder einen Aralkylrest mit 7 bis 20 Kohlenstoffatomen bedeutet, und 1,2-Diolen der Formel

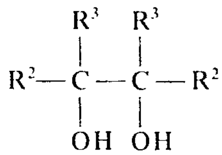

(II)

worin die einzelnen Reste $R^2$ und $R^3$ gleich oder verschieden sind und jeweils ein Wasserstoffatom oder einen aliphatischen Rest bezeichnen, das Restepaar $R^3$ auch zusammen mit den beiden benachbarten Kohlenstoffatomen für die Glieder eines Ringes stehen können, dadurch gekennzeichnet, daß man cyclische, aliphatische Carbonate der Formel

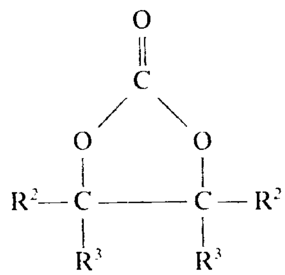

(III)

worin $R^2$ und $R^3$ die vorgenannte Bedeutung besitzen, mit Aminen der Formel

$$R^1—NH_2 \qquad (IV)$$

worin $R^1$ die vorgenannte Bedeutung besitzt, in einem Verhältnis von 2 bis 20 Mol Ausgangsstoff IV je Mol Ausgangsstoff III bei einer Temperatur oberhalb 100° C umsetzt.

**Claim**

A process for the simultaneous preparation of 1,3-disubstituted ureas of the formula

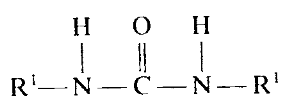

(I)

where $R^1$ is alkyl of 1 to 20 carbon atoms, which may be substituted by phenoxy groups and/or alkoxy groups of 1 to 13 carbon atoms, monocycloalkyl of 3 to 8 carbon atoms, bicycloalkyl of 6 to 12 carbon atoms or aralkyl of 7 to 20 carbon atoms, and 1,2-diols of the formula

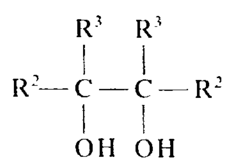

(II)

where the individual radicals $R^2$ and $R^3$ are identical or different and each is hydrogen or an aliphatic

radical, and the two radicals $R^3$ together with the two adjacent carbon atoms can also be members of a ring, characterized in that cyclic, aliphatic carbonates of the formula

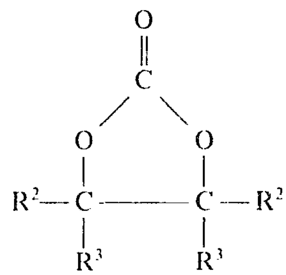

(III)

where $R^2$ and $R^3$ have above meanings, are reacted with amines of the formula

$$R^1—NH_2 \quad (IV)$$

where $R^1$ has the above meaning, in a ratio of from 2 to 20 moles of starting material IV per mole of starting material III at a temperature above 100° C.

**Revendication**

Procédé pour préparer simultanément des urées 1,3-disubstituées, de formule

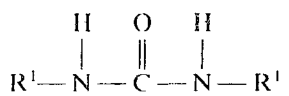

(I)

dans laquelle $R^1$ représente un groupe alkyle en C1—C20 qui peut être substitué par des groupes phénoxy et/ou des groupes alcoxy en C1—C13, un groupe monocycloalkyle en C3—C8, un groupe bicycloalkyle en C6—C12 ou un groupe aralkyle en C7—C20, et des 1,2-diols de formule

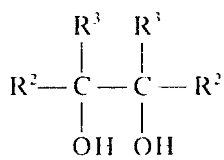

(II)

dans laquelle les divers symboles $R^2$ et $R^3$, ayant des significations identiques ou différentes, représentent chacun un atome d'hydrogène ou un groupe aliphatique, les deux symboles $R^3$ pouvant également représenter, ensembłe et avec les deux atomes decarbone voisins, des chaînons d'un cycle, caractérisé en ce que l'on fait réagir des carbonates aliphatiques cycliques de formule

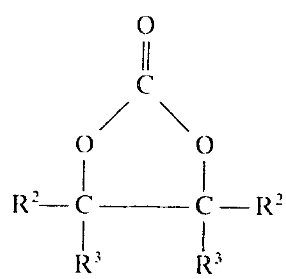

(III)

dans laquelle $R^2$ et $R^3$ ont les significations indiquées ci-dessus, avec des amines de formule

$$R^1—NH_2 \quad (IV)$$

dans laquelle $R^1$ a les significations indiquées ci-dessus, dans des proportions relatives de 2 à 20 moles du composant de départ IV par mole du composant de départ III à une température supérieure à 100° C.